# EUROPEAN PATENT APPLICATION

(11) **EP 4 261 223 A1**
(43) Date of publication of application: **18.10.2023**
(21) Application number: 21902685.3
(22) Date of filing: 09.12.2021
(51) Int. Cl.: C07K 16/28, C12P 21/02, A61K 39/395, A61P 35/00

(54) **ANTI-SIRP? ANTIBODY AND APPLICATION THEREOF**

(30) Priority: 11.12.2020 CN 202011464010
(71) Applicant: BioRay Pharmaceutical Co., Ltd., Jiaojiang District, Taizhou City, Zhejiang (CN); Hisun Biopharmaceutical Co., Ltd., zhejiang province 311404 (CN)
(72) Inventor: WU, Zhenhua, Taizhou, Zhejiang 318000 (CN); NIE, Lei, Taizhou, Zhejiang 318000 (CN); MEI, Xiaofen, Taizhou, Zhejiang 318000 (CN); WANG, Haibin, Taizhou, Zhejiang 318000 (CN); CHEN, Juan, Taizhou, Zhejiang 318000 (CN); LI, Na, Taizhou, Zhejiang 318000 (CN); WANG, Xiaoze, Taizhou, Zhejiang 318000 (CN); ZHOU, Yaqiong, Taizhou, Zhejiang 318000 (CN); CHEN, Yao, Taizhou, Zhejiang 318000 (CN); JIANG, Meizhu, Taizhou, Zhejiang 318000 (CN)
(74) Representative: Kador & Partner Part mbB
(86) International application number: PCT/CN2021/136763
(87) International publication number: WO 2022/121980

(57) **Abstract**

The present invention provides an anti-SIRPα antibody and an antigen binding fragment thereof, being capable of effectively blocking the binding of SIRPα and CD47, thereby effectively promoting the phagocytosis of macrophages to tumor cells, effectively inhibiting the growth of tumors, and having a good pharmaceutical prospect.

## Description

### TECHNICAL FIELD

The present invention belongs to the field of biology, and relates to an anti-SIRPα antibody.

### BACKGROUND

SIRPα is a transmembrane protein expressed on the surface of myeloid cells such as macrophages, monocytes, dendritic cells, granulocytes, and is a member of the immunoglobulin superfamily (IgSF). SIRPα is composed of an N-terminal extracellular region, a transmembrane region and a C-terminal intracellular region. The extracellular region comprises three IgSF domains (the N terminus is an Ig-V domain), and the intracellular region comprises an immunoreceptor tyrosine-based inhibitory motif (ITIM). The major ligand of SIRPα is CD47, which is a group of transmembrane glycoproteins that are widely expressed in normal tissues and lesions. The binding of CD47 to SIRPα on myeloid cells such as macrophages leads to the phosphorylation of ITIM in the SIRPα intracellular region, which in turn recruits and activates the phosphatases SHP-1 and SHP-2, thereby inhibiting the phagocytosis of macrophages through downstream signaling. Normal tissues release the "don't eat me" signal as a self-protection mechanism by expressing CD47 and inducing CD47-SIRPα signaling.

It has been found that CD47 is overexpressed on almost all tumor cells. Tumor cells escape from the surveillance of immune cells by sending a "don't eat me" signal through the binding of CD47 to SIRPα. Clinically, CD47 expression levels are closely related to poor prognosis of patients. *In vitro* and *in vivo* studies have also demonstrated that blocking CD47/SIRPα can promote phagocytosis of tumor cells and inhibit tumor growth in animal models. The above shows that targeting CD47/SIRPα can be used as a new approach for developing tumor immunotherapies. Considering that CD47 is widely expressed on normal cells or tissues, particularly on red blood cells and platelets, targeting CD47 may cause hematologic toxicity. Besides, in addition to SIRPα, CD47 could also interact with TSP-1 and integrin. CD47 is involved in various signalling pathways, and targeting CD47 may lead to many safety concerns. Therefore, developing anti-SIRPα antibodies to block the CD47/SIRPα signaling pathway may be a more effective strategy for cancer treatment.

### SUMMARY

The present invention is intended to provide a new anti-SIRPα antibody, which can specifically bind to SIRPα and has great potential for tumor treatment.

In a first aspect, the present invention provides an anti-SIRPα antibody that binds to SIRPα or a fragment thereof, or an antigen-binding fragment thereof. The antibody or the antigen-binding fragment thereof comprises a heavy chain variable region comprising three CDRs, VH CDR1, VH CDR2 and VH CDR3, and a light chain variable region comprising three CDRs, VL CDR1, VL CDR2 and VL CDR3; wherein
the VH CDR1 comprises or consists of an amino acid sequence set forth in SEQ ID NO: 3, 13, 23 or 33, the VH CDR2 comprises or consists of an amino acid sequence set forth in SEQ ID NO: 4, 14, 24 or 34, the VH CDR3 comprises or consists of an amino acid sequence set forth in SEQ ID NO: 5, 15, 25 or 35, the VL CDR1 comprises or consists of an amino acid sequence set forth in SEQ ID NO: 8, 18, 28 or 38, the VL CDR2 comprises or consists of an amino acid sequence set forth in SEQ ID NO: 9, 19, 29 or 39, and the VL CDR3 comprises or consists of an amino acid sequence set forth in SEQ ID NO: 10, 20, 30 or 40.

In preferred embodiments, the antibody comprises VH CDR1, VH CDR2 and VH CDR3, and VL CDR1, VL CDR2 and VL CDR3, wherein
the amino acid sequence of the VH CDR1 is set forth in SEQ ID NO: 3;
the amino acid sequence of the VH CDR2 is set forth in SEQ ID NO: 4;
the amino acid sequence of the VH CDR3 is set forth in SEQ ID NO: 5;
the amino acid sequence of the VL CDR1 is set forth in SEQ ID NO: 8;
the amino acid sequence of the VL CDR2 is set forth in SEQ ID NO: 9; and
the amino acid sequence of the VL CDR3 is set forth in SEQ ID NO: 10.

In preferred embodiments, the antibody comprises VH CDR1, VH CDR2 and VH CDR3, and VL CDR1, VL CDR2 and VL CDR3, wherein
the amino acid sequence of the VH CDR1 is set forth in SEQ ID NO: 13;
the amino acid sequence of the VH CDR2 is set forth in SEQ ID NO: 14;
the amino acid sequence of the VH CDR3 is set forth in SEQ ID NO: 15;
the amino acid sequence of the VL CDR1 is set forth in SEQ ID NO: 18;
the amino acid sequence of the VL CDR2 is set forth in SEQ ID NO: 19; and
the amino acid sequence of the VL CDR3 is set forth in SEQ ID NO: 20.

In preferred embodiments, the antibody comprises VH CDR1, VH CDR2 and VH CDR3, and VL CDR1, VL CDR2 and VL CDR3, wherein
the amino acid sequence of the VH CDR1 is set forth in SEQ ID NO: 23;
the amino acid sequence of the VH CDR2 is set forth in SEQ ID NO: 24;
the amino acid sequence of the VH CDR3 is set forth in SEQ ID NO: 25;
the amino acid sequence of the VL CDR1 is set forth in SEQ ID NO: 28;
the amino acid sequence of the VL CDR2 is set forth in SEQ ID NO: 29; and
the amino acid sequence of the VL CDR3 is set forth in SEQ ID NO: 30.

In preferred embodiments, the antibody comprises VH CDR1, VH CDR2 and VH CDR3, and VL CDR1, VL CDR2 and VL CDR3, wherein
the amino acid sequence of the VH CDR1 is set forth in SEQ ID NO: 33;
the amino acid sequence of the VH CDR2 is set forth in SEQ ID NO: 34;
the amino acid sequence of the VH CDR3 is set forth in SEQ ID NO: 35;
the amino acid sequence of the VL CDR1 is set forth in SEQ ID NO: 38;
the amino acid sequence of the VL CDR2 is set forth in SEQ ID NO: 39; and
the amino acid sequence of the VL CDR3 is set forth in SEQ ID NO: 40.

In some embodiments, the amino acid sequence of the heavy chain variable region is set forth in SEQ ID NO: 1, 11, 21 or 31, or has at least 90%, at least 95%, at least 96%, at least 97%, at least 98% or at least 99% sequence identity thereto.

In some embodiments, the amino acid sequence of the light chain variable region is set forth in SEQ ID NO: 6, 16, 26 or 36, or has at least 90%, at least 95%, at least 96%, at least 97%, at least 98% or at least 99% sequence identity thereto.

In certain preferred embodiments, the three CDRs in the heavy chain variable region and/or the three CDRs in the light chain variable region are defined according to the Kabat or Chothia numbering system.

In some embodiments, the antibody or the antigen-binding fragment thereof disclosed herein may further comprise one or more of a heavy chain constant region, a light chain constant region and an Fc region. In further preferred embodiments, the light chain constant region is a λ chain or a κ chain constant region. In some preferred embodiments, the antibody or the antigen-binding fragment thereof is of IgG1, IgG2, IgG3 or IgG4 type.

In some embodiments, the antibody or the antigen-binding fragment thereof is a chimeric antibody or a humanized antibody, or an antigen-binding fragment thereof.

In a second aspect, provided is a nucleic acid molecule comprising nucleotides encoding the antibody or the antigen-binding fragment thereof disclosed herein. In some embodiments, the nucleic acid molecule encodes the heavy chain variable region and/or the light chain variable region of the antibody or the antigen-binding fragment thereof.

In preferred embodiments, the nucleic acid molecule encodes the heavy chain variable region, and the corresponding nucleotide sequence is set forth in SEQ ID NO: 2, 12, 22 or 32, or has at least 90%, at least 95%, at least 96%, at least 97%, at least 98% or at least 99% sequence identity thereto. In other preferred embodiments, the nucleic acid molecule encodes the light chain variable region, and the corresponding nucleotide sequence is set forth in SEQ ID NO: 7, 17, 27 or 37, or has at least 90%, at least 95%, at least 96%, at least 97%, at least 98% or at least 99% sequence identity thereto.

In a third aspect, the present invention provides a biomaterial, which is:
(1) a vector, a host cell, a microorganism or the like comprising the nucleic acid molecule disclosed herein; or
(2) an expression product, a suspension, a supernatant or the like of the above (1).

Those skilled in the art can easily select and prepare vectors, host cells or microorganisms comprising the coding sequence of the antibody according to the amino acid sequence of the antibody, and can be aware of how to culture such host cells or microorganisms to obtain the corresponding expression products, suspensions, supernatants, etc., so as to obtain the corresponding antibody. These are all conventional techniques in the art.

In a fourth aspect, provided is a composition comprising the antibody or the antigen-binding fragment thereof disclosed herein; preferably, the composition is a pharmaceutical composition further comprising a pharmaceutically acceptable carrier.

In a fifth aspect, provided is a method for preparing the antibody or the antigen-binding fragment thereof disclosed herein, comprising: culturing the above host cell to express the antibody or the antigen-binding fragment, and isolating the antibody or the antigen-binding fragment.

In a sixth aspect, provided is use of the antibody or the antigen-binding fragment thereof, the nucleic acid molecule, the biomaterial or the composition disclosed herein in preparing a medicament for treating a tumor; preferably, the tumor is a CD47-positive tumor; more preferably, the tumor is a hematological tumor or a solid tumor, such as leukemia, lymphoma, bladder cancer, breast cancer, head and neck cancer, gastric cancer, melanoma, pancreatic cancer, colorectal cancer, esophageal cancer, liver cancer, kidney cancer, lung cancer, prostate cancer, ovarian cancer, thyroid cancer, neuroglioma and other solid tumors.

In a seventh aspect, provided is use of the antibody or the antigen-binding fragment thereof, the nucleic acid molecule, the biomaterial or the composition disclosed herein in preparing a formulation that blocks the binding of SIRPα to CD47.

In an eighth aspect, provided is use of the antibody or the antigen-binding fragment thereof, the nucleic acid molecule, the biomaterial or the composition disclosed herein in combination with one or more additional cancer therapeutic agents in preparing a medicament for treating a tumor; preferably, the tumor is a CD47-positive tumor.

In some preferred embodiments, the additional cancer therapeutic agent includes, but is not limited to, a chemotherapeutic agent, a radiotherapeutic agent and a biomacromolecule drug. Further preferably, the biomacromolecule drug is a monoclonal antibody drug targeting a tumor cell surface antigen, including an anti-CD20 antibody (such as zuberitamab or rituximab), cetuximab and trastuzumab.

In a ninth aspect, provided is a method for treating a tumor, wherein the tumor is a CD47-positive tumor; preferably, the tumor is a hematological tumor or a solid tumor, such as leukemia, lymphoma, bladder cancer, breast cancer, head and neck cancer, gastric cancer, melanoma, pancreatic cancer, colorectal cancer, esophageal cancer, liver cancer, kidney cancer, lung cancer, prostate cancer, ovarian cancer, thyroid cancer, neuroglioma and other solid tumors; the method comprises administering to an individual in need the antibody or the antigen-binding fragment thereof, the nucleic acid molecule, the biomaterial or the composition disclosed herein.

In a tenth aspect, provided is a method for blocking the binding of SIRPα to CD47, comprising using the antibody or the antigen-binding fragment thereof, the nucleic acid molecule, the biomaterial or the composition disclosed herein.

In an eleventh aspect, provided is a method for treating a tumor with a combination therapy, wherein the tumor is a CD47-positive tumor; the method comprises administering to an individual in need the antibody or the antigen-binding fragment thereof, the nucleic acid molecule, the biomaterial or the composition disclosed herein, and one or more additional cancer therapeutic agent; preferably, the additional cancer therapeutic agent includes, but is not limited to, a chemotherapeutic agent, a radiotherapeutic agent and a biomacromolecule drug; more preferably, the biomacromolecule drug is a monoclonal antibody drug targeting a tumor cell surface antigen, including an anti-CD20 antibody (such as zuberitamab or rituximab), cetuximab and trastuzumab.

The anti-SIRPα antibody or the antigen-binding fragment thereof disclosed herein is a blocking antibody, which is capable of effectively blocking the binding of SIRPα to CD47, thereby effectively promoting the phagocytosis of macrophage to tumor cells, effectively inhibiting the growth of tumors, and having a good pharmaceutical prospect.

### BRIEF DESCRIPTION OF THE DRAWINGS

FIG. 1 shows the blocking effect of murine monoclonal antibodies on the binding of SIRPα to CD47.
FIG. 2 shows the binding of humanized monoclonal antibodies to SIRPα.
FIG. 3 shows the blocking effect of humanized monoclonal antibodies on the binding of SIRPα to CD47.
FIG. 4A shows the blocking effect of #14 antibody and KWAR23 on the binding of SIRPα (V1) to CD47.
FIG. 4B shows the blocking effect of #14 antibody, 18D5 and 1H9 on the binding of SIRPα (V1) to CD47.
FIG. 4C shows the blocking effect of #14 antibody, KWAR23, 18D5 and 1H9 on the binding of SIRPα (V2) to CD47.
FIG. 4D shows the blocking effect of #14 antibody, KWAR23, 18D5 and 1H9 on the binding of SIRPα (V8) to CD47.
FIG. 5 shows the binding kinetics of #14 antibody and KWAR23 to a recombinant SIRPα protein.
FIG. 6A shows the promotion of anti-CD20 antibody-induced phagocytosis by #14 antibody; FIG. 6B shows the promotion of anti-HER2 antibody-induced phagocytosis by #14 antibody.
FIG. 7 shows the anti-tumor efficacy of #14 antibody in an MC38 tumor model.
FIG. 8 shows that #14 antibody and the anti-CD20 antibody can synergistically inhibit tumor growth.

### DETAILED DESCRIPTION

The present invention will be illustrated with reference to the following specific examples. The reagents and instruments used in the following procedures are those conventional in the art and commercially available, unless otherwise specified; the methods used are all conventional in the art, and those skilled in the art can undoubtedly implement the methods and obtain the corresponding results according to the description of the examples.

### Definitions:

In the present invention, the term "antibody" is an immunoglobulin that can specifically recognize and bind to an antigen, and encompasses a variety of antibody structures, including but not limited to, monoclonal antibodies, polyclonal antibodies, bispecific antibodies or antibody fragments, synthesized antibodies, antibodies recombinantly produced, human antibodies, non-human antibodies (e.g., murine antibodies), humanized antibodies, chimeric antibodies, and intracellular antibodies and antibody fragments, such as but not limited to, Fab fragments, Fab' fragments, F(ab')² fragments, Fv fragments, Fd fragments, Fd' fragments, single-chain Fv (scFv), single-chain Fab (scFab), or antigen-binding fragments of any of the above antibodies. The antibodies provided herein include members of any class (e.g., IgG, IgM, IgD, IgE, IgA, or IgY), any subclass (e.g., IgG1, IgG2, lgG3, IgG4, IgA1, or IgA2) or type (e.g., IgG2a or IgG2b) of immunoglobulin. In preferred embodiments, the antibody disclosed herein is a murine antibody, a chimeric antibody or a humanized antibody.

As used herein, an "antibody fragment" or an "antigen-binding fragment" of an antibody refers to any portion of a full-length antibody that is less than full length, but comprises at least a portion of the variable region of the antibody that binds to the antigen (e.g., one or more CDRs and/or one or more binding sites of the antibody), and thus retains the binding specificity as well as at least a portion of the specific binding capacity of the full-length antibody. Thus, an antigen-binding fragment refers to an antibody fragment that comprises an antigen-binding portion that binds to the same antigen as the antibody from which the antibody fragment is derived. The antibody fragments include antibody derivatives produced by enzyme treatment of full-length antibodies, as well as synthesized derivatives, e.g., recombinantly produced derivatives. The antibodies include antibody fragments. Examples of antibody fragments include, but are not limited to, Fab, Fab', F(ab')2, single-chain Fv (scFv), Fv, dsFv, diabodies, Fd and Fd' fragments, and other fragments, including modified fragments (see, e.g., Methods in Molecular Biology, Vol 207: Recombinant Antibodies for Cancer Therapy Methods and Protocols (2003); Chapter 1; p 3-25, Kipriyanov). The fragment may comprise multiple chains linked together, for example, by a disulfide bond and/or by a peptide linker. The antibody fragment generally comprises at least or about 50 amino acids, and typically at least or about 200 amino acids. The antigen-binding fragment includes any antibody fragment that, when inserted into an antibody framework (e.g., by replacing the corresponding region), yields an antibody that immunospecifically binds to an antigen.

As used herein, a "conventional antibody" refers to an antibody comprising two heavy chains (which may be designated H and H'), two light chains (which may be designated L and L') and two antigen-binding sites, wherein each heavy chain may be a full-length immunoglobulin heavy chain or any functional region thereof that retains the antigen-binding capability (e.g., the heavy chain includes but is not limited to VH chain, VH-CH1 chain and VH-CH1-CH2-CH3 chain), and each light chain may be a full-length light chain or any functional region thereof (e.g., the light chain includes but is not limited to VL chain and VL-CL chain). Each heavy chain (H or H') is paired with one light chain (L or L', respectively).

As used herein, a full-length antibody is an antibody having two full-length heavy chains (e.g., VH-CH1-CH2-CH3) and two full-length light chains (VL-CL) and a hinge region, e.g., an antibody naturally produced by an antibody-secreting B cell and a synthetically produced antibody having the same domains.

As used herein, dsFv refers to Fv with an engineered intermolecular disulfide bond that stabilizes the VH-VI, pair.

As used herein, an Fab fragment is an antibody fragment obtained by digesting a full-length immunoglobulin with papain, or a fragment of the same structure produced synthetically, e.g., by a recombinant method. The Fab fragment comprises a light chain (comprising VL and CL) and another chain comprising the variable domain of the heavy chain (VH) and one constant domain of the heavy chain (CH1).

As used herein, an F(ab')2 fragment is an antibody fragment obtained by digesting an immunoglobulin with pepsin at pH 4.0-4.5, or a fragment of the same structure produced synthetically, e.g., by a recombinant method. The F(ab')2 fragment substantially comprises two Fab fragments, wherein each heavy chain moiety comprises a few additional amino acids, including cysteines that form a disulfide bond linking the two fragments.

As used herein, an Fab' fragment is a fragment that comprises half of an F(ab')2 fragment (one heavy chain and one light chain).

As used herein, an ScFv fragment refers to an antibody fragment comprising a light chain variable region (VL) and a heavy chain variable region (VH) covalently linked in any order by a polypeptide linker.

The term "variable region" refers to a domain of an antibody heavy chain or light chain that recognizes and specifically binds to an antigen epitope, which comprises an amino acid sequence that varies among different antibodies. Each light chain or each heavy chain has a variable region domain VL or VH, respectively. The variable domains provide antigen specificity and are therefore responsible for antigen recognition. Each variable region comprises CDRs and framework regions (FRs) and the CDRs are parts of the antigen-binding site domain.

A CDR region or a "complementarity determining region" refers to a region in an antibody variable region that is hypervariable in sequence and forms a structurally defined loop and/or comprises antigen-contacting amino acid residues. CDRs are primarily responsible for the binding of the antibody to an antigen epitope, and determine the specificity of the antibody. In a given amino acid sequence of a heavy chain variable region or a light chain variable region, the specific amino acid sequences of the CDRs are determined using any one or a combination of many well-known numbering schemes including, e.g., Kabat, Contact, AbM and Chothia. The CDRs of the antibody of the present invention can be determined according to any scheme in the art or a combination thereof.

As used herein, "hypervariable region", "complementarity determining region" and "CDR" can be used interchangeably and refer to one of a plurality of portions within each variable region that jointly form the antigen-binding sites of the antibody. Each variable region domain comprises three CDRs, designated CDR1, CDR2 and CDR3. For example, the light chain variable region domain comprises three CDRs, designated VL CDR1, VL CDR2 and VL CDR3; the heavy chain variable region domain comprises three CDRs, designated H CDR1 (or VH CDR1), H CDR2 (or VH CDR2) and H CDR3 (or VH CDR3). The three CDRs in the variable region are not contiguous along the linear amino acid sequence, but are close in the folded polypeptide. The CDRs are located within loops of parallel chains that connect the β-sheet of the variable domain. As described herein, the CDRs are known to those skilled in the art and can be identified based on Kabat or Chotnia numbering by those skilled in the art (see, e.g., Kabat, E.A. et al., (1991) Sequences of Proteins of Immunological Interest, Fifth Edition, U.S. Department of Health and Human Services, AIH Publication No. 91-3242, and Chothia, C. et al. (1987) J. Mol. Biol. 196:901-917).

As used herein, a framework region (FR) is a domain within the variable region domain of an antibody that is located within the β-sheet; the FR regions are more conserved relative to the hypervariable regions in terms of the amino acid sequence.

As used herein, a "constant region" domain is a domain in a heavy chain or a light chain of an antibody, which comprises an amino acid sequence that is more conserved relative to the amino acid sequence of the variable region domain. In conventional full-length antibody molecules, each light chain has a single light chain constant region (CL) domain, and each heavy chain comprises one or more heavy chain constant region (CH) domains, including CH1, CH2, CH3 and CH4. The antibody constant region may serve effector functions such as, but not limited to, eliminating antigens, pathogens and toxins to which the antibody specifically binds, for example, by interacting with various cells, biomolecules and tissues.

As used herein, a functional region of a VH domain is at least a portion of an intact VH domain that retains at least part of the binding specificity of the intact VH domain (e.g., by retaining one or more CDRs of the intact VH domain), such that the functional region of the VH domain binds to an antigen alone or in combination with another antibody domain (e.g., a VL domain) or a region thereof. Exemplary functional regions of VH domains are regions comprising the CDR1, CDR2 and/or CDR3 of the VH domains.

As used herein, a functional region of a VL domain is at least a portion of an intact VL domain that retains at least part of the binding specificity of the intact VL domain (e.g., by retaining one or more CDRs of the intact VL domain), such that the functional region of the VL domain binds to an antigen alone or in combination with another antibody domain (e.g., a VH domain) or a region thereof. Exemplary functional regions of VL domains are regions comprising the CDR1, CDR2 and/or CDR3 of the VL domains.

As used herein, the terms "polynucleotide" and "nucleic acid molecule" refer to an oligomer or a polymer comprising at least two linked nucleotides or nucleotide derivatives, including deoxyribonucleic acid (DNA) and ribonucleic acid (RNA) that are typically linked together by phosphodiester bonds.

As used herein, an isolated nucleic acid molecule is a nucleic acid molecule that is isolated from other nucleic acid molecules present in the natural source of the nucleic acid molecules. An "isolated" nucleic acid molecule, such as a cDNA molecule, can be substantially free of other cellular material or culture medium when prepared by recombinant techniques, or substantially free of chemical precursors or other chemical components when chemically synthesized. Exemplary isolated nucleic acid molecules provided herein include isolated nucleic acid molecules encoding the antibodies or the antigen-binding fragments provided herein.

The sequence "identity" has a meaning well known in the art, and a percentage sequence identity between two nucleic acid molecules, peptide molecules or regions can be calculated using the disclosed techniques in the art. The sequence identity can be measured along the entire length of a polynucleotide or a polypeptide or along a region of the molecule. (See, e.g., Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H. G., eds., Humana Press, New Jersey, 1994; Sequence Analysis in Molecular Biology, von Heinj e, G., Academic Press, 1987; and Sequence Analysis Primer, Gribskov, M. and Devereux, J., eds., M Stockton Press, New York, 1991). Although there are many methods for measuring the identity between two polynucleotides or polypeptides, the term "identity" is well known to those skilled (Carrillo, H. & Lipman, D., SIAM J Applied Math 48:1073 (1988)).

As used herein, "expression" refers to the process of producing a polypeptide by the transcription and translation of a polynucleotide. The expression level of a polypeptide can be evaluated using any method known in the art, including, for example, methods for determining the amount of a polypeptide produced in a host cell. Such methods may include, but are not limited to, quantification of polypeptides in cell lysates by ELISA, Coomassie blue staining after gel electrophoresis, Lowry protein assay and Bradford protein assay.

As used herein, a "host cell" is a cell that is used to receive, maintain, replicate and amplify a vector. The host cell can also be used to express a polypeptide encoded by the vector. When the host cell divides, the nucleic acid in the vector replicates, and is thus amplified. The host cell can be a eukaryotic cell or a prokaryotic cell. Suitable host cells include, but are not limited to, CHO cells, HeLa cells and HEK cells such as HEK 293 cells.

As used herein, a "vector" is a replicable nucleic acid from which one or more heterologous proteins may be expressed when the vector is transformed into an appropriate host cell. The vectors include those into which a nucleic acid encoding a polypeptide or a fragment thereof may be introduced, typically by restriction enzyme digestion and ligation. The vectors also include those comprising a nucleic acid encoding a polypeptide. The vectors are used to introduce the nucleic acid encoding the polypeptide into host cells for amplifying the nucleic acid or for expressing/displaying the polypeptide encoded by the nucleic acid. The vectors are usually in a free state, but can be designed to integrate a gene or a portion thereof into chromosomes of the genome. Artificial chromosomal vectors, such as yeast artificial vectors and mammalian artificial chromosomes, are also contemplated. The selection and use of such vehicles are well known to those skilled in the art.

As used herein, an "expression vector" includes vectors capable of expressing DNA operably linked to regulatory sequences, such as promoter regions, capable of affecting the expression of such DNA fragments. Such additional fragments may include promoter and terminator sequences, and optionally may include one or more origins of replication, one or more selection markers, enhancers, polyadenylation signals, and the like. The expression vectors are generally derived from plasmids or viral DNA, or may comprise their elements. Thus, an expression vector refers to a recombinant DNA or RNA construct, such as a plasmid, a phage, a recombinant virus, or other vectors that, when introduced into an appropriate host cell, cause the expression of the cloned DNA. Suitable expression vectors are well known to those skilled in the art and include expression vectors replicable in eukaryotic cells and/or prokaryotic cells as well as expression vectors that maintain a free state or expression vectors that are integrated into the genome of the host cell.

As used herein, "treating" an individual having a disease or a condition means that the individual's symptoms are partially or fully alleviated, or remain unchanged after the treatment. Thus, the treatment includes prevention, treatment and/or cure. The prevention refers to prevention of the underlying diseases and/or prevention of the worsening of the symptoms or disease progression. The treatment also includes any pharmaceutical use of any antibody or antigen-binding fragment thereof as well as the composition provided herein.

As used herein, "efficacy" means an effect resulting from the treatment of an individual that alters, typically ameliorates or improves symptoms of a disease or a condition, or cures a disease or a condition.

As used herein, "therapeutically effective amount" or "therapeutically effective dose" refers to an amount of a substance, a compound, a material, or a composition comprising a compound that is at least sufficient to produce a therapeutic effect after being administered to a subject. Thus, it is the amount necessary to prevent, cure, improve, arrest, or partially arrest the symptoms of the disease or the condition. Also, as used herein, a "prophylactically effective amount" or a "prophylactically effective dose" refers to an amount of a substance, a compound, a material or a composition comprising a compound that, when administered to a subject, will possess the desired prophylactic effect, e.g., preventing or delaying the occurrence or recurrence of a disease or a condition, or reducing the possibility of the occurrence or recurrence of a disease or a condition. A complete prophylactically effective dose is not necessarily reached by the administration of one dose, but may be reached after the administration of a series of doses. Thus, a prophylactically effective amount may be administered through one or more doses.

As used herein, the term "individual" refers to a mammal, such as a human.

### Anti-SIRPα antibody disclosed herein

The present invention provides an anti-SIRPα antibody having high affinity for human SIRPα protein. The antibody can effectively inhibit the binding of SIRPα to its ligand CD47, thereby blocking the signaling downstream to CD47/SIRPα, promoting the phagocytosis of tumor cells by macrophages, and further realizing the elimination of tumor cells. When binding to SIRPα protein on a cell surface, the anti-SIRPα antibody described herein can cause endocytosis, resulting in reduced expression of SIRPα protein on cell surface, thereby further reducing CD47/SIRPα signaling.

The anti-SIRPα antibody or the antigen-binding fragment thereof disclosed herein comprises a substitution, insertion or deletion. The anti-SIRPα antibody disclosed herein comprises a modification to a light chain variable region, a heavy chain variable region, a light chain or a heavy chain, and the modified amino acid sequence thereof is different from the amino acid sequence from which the antibody is derived. For example, an amino acid sequence derived from the same given protein may be similar to the initial sequence, e.g., have a certain percent identity. For example, it may have 60%, 65%, 70%, 75%, 80%, 85%, 90%, 95%, 98% or 99% identity to the initial sequence.

As used herein, "identity" refers to, when sequences of two peptides or two nucleic acid molecules are aligned, the percent of identical bases (or amino acids) in the two compared sequences. Such alignment and the percentage homology or sequence identity can be determined using software programs known in the art, such as those described in Ausubel et al., eds., (2007), Current Protocols in Molecular Biology*.* Preferably, the alignment is performed using default parameters. One alignment program is BLAST using default parameters. In particular, the program is BLASTN or BLASTP.

In certain embodiments, amino acid modifications, which may be one or more, may be introduced into the Fc region of the antibody provided herein, thereby generating an Fc variant. The Fc variant may comprise a human Fc region sequence comprising an amino acid modification at one or more amino acid positions.

Suitable "antibodies and antigen-binding fragments thereof" in the present invention include, but are not limited to, polyclonal, monoclonal, monovalent, bispecific, multispecific, recombinant, heterologous, chimeric, humanized or de-immunogenic antibodies, or Fab fragments, Fab' fragments, F(ab')2 fragments, single chain antibodies, nanobodies, and epitope-binding fragments of any of the foregoing.

In some embodiments, the antibody disclosed herein may be monospecific, bispecific or multispecific. The anti-SIRPα antibody may be ligated to another antibody or antibody fragment to produce a bispecific or multispecific antibody with a second or more binding specificities.

In certain embodiments, the antibody may be further modified to add functional components. Suitable moieties for antibody derivatization include, but are not limited to, PEG, dextran, proteins, lipids, therapeutic agents and toxins. The antibody may be modified by phosphorylation, acetylation, glycosylation, pegylation, amidation, ligation to other proteins, or the like.

### Tumor treatment and use of the antibody

The anti-SIRPα antibody and the antigen-binding fragment thereof and the pharmaceutical composition comprising the same provided herein can be used for diagnosing, prognosing, treating or inhibiting cancers. The present invention relates to a method for treating a cancer in a subject by administering the anti-SIRPα antibody or the fragment thereof disclosed herein to a subject in need. The therapeutic compounds described herein include, but are not limited to, the antibodies disclosed herein (including the variants and derivatives disclosed herein) and nucleic acids or polynucleotides encoding the antibodies disclosed herein (including the variants and derivatives disclosed herein).

The present invention further provides a combination therapy comprising combined use of the anti-SIRPα antibody disclosed herein and at least one additional therapeutic agent including, but not limited to, a chemotherapeutic agent, a radiotherapeutic agent and a biomacromolecule drug. In one embodiment, the biomacromolecule drug is a monoclonal antibody drug targeting a tumor cell surface antigen, including an anti-CD20 antibody (such as zuberitamab or rituximab), cetuximab and trastuzumab.

The antibody disclosed herein (and any additional therapeutic agents) may be administered through any suitable means, including, but not limited to, intraperitoneal, intravenous, subcutaneous, intranasal and intramuscular injection. The antibody and the variant or the composition thereof may be administered by any convenient route, for example, by bolus injection or infusion, or absorption through epithelium or skin mucous membrane.

### Anti-SIRPα antibody sequences exemplified herein

**Table 1: SEQ ID NOs. of VH, VH-CDR1, VH-CDR2, VH-CDR3, VL, VL-CDR1, VL-CDR2 and VL-CDR3 amino acid sequences of the antibodies disclosed herein**

| Antibody No. | VH SEQ ID NO: | VH-CDR1 SEQ ID NO: | VH-CDR2 SEQ ID NO: | VH-CDR3 SEQ ID NO: | VL SEQ ID NO: | VL-CDR1 SEQ ID NO: | VL-CDR2 SEQ ID NO: | VL-CDR3 SEQ ID NO: |
|---|---|---|---|---|---|---|---|---|
| #4 | 1 | 3 | 4 | 5 | 6 | 8 | 9 | 10 |
| #11 | 11 | 13 | 14 | 15 | 16 | 18 | 19 | 20 |
| #13 | 21 | 23 | 24 | 25 | 26 | 28 | 29 | 30 |
| #14 | 31 | 33 | 34 | 35 | 36 | 38 | 39 | 40 |

**Table 2: SEQ ID NOs. of VH and VL DNA sequences of the antibodies disclosed herein**

| Antibody No. | VH DNA SEQ ID NO: | VL DNA SEQ ID NO: |
|---|---|---|
| #4 | 2 | 7 |
| #11 | 12 | 17 |
| #13 | 22 | 27 |
| #14 | 32 | 37 |

**Table 3: Sequences of the present invention**

| SEQ ID NO: | Name | Sequence |
|---|---|---|
| 1 | #4 VH | |
| 2 | #4 VH DNA | EVQLVQSGAEVKKPGATVKISCRGSGFNIKDYYIQWVQQA PGKGLEWMGWIDPENGDTKYAPKFQGRVTITADTSTDTAY MELSSLRSEDTAVYYCYAKGPYWGQGTTLTVSS GAGGTGCAGCTGGTGCAGTCCGGCGCCGAGGTGAAGAA GCCCGGCGCCACCGTGAAGATCTCCTGCCGCGGCTCCGG CTTCAACATCAAGGACTACTACATCCAGTGGGTGCAGCA GGCCCCCGGCAAGGGCCTGGAGTGGATGGGCTGGATCG ACCCCGAGAACGGCGACACCAAGTACGCCCCCAAGTTC CAGGGCCGCGTGACCATCACCGCCGACACCTCCACCGAC ACCGCCTACATGGAGCTGTCCTCCCTGCGCTCCGAGGAC ACCGCCGTGTACTACTGCTACGCCAAGGGCCCCTACTGG GGCCAGGGCACCACCCTGACCGTCTCGAGC |
| 3 | #4 VH-CDR1 | DYYIQ |
| 4 | #4 VH-CDR2 | WIDPENGDTKYAPKFQG |
| 5 | #4 VH-CDR3 | KGPY |
| 6 | #4 VL | |
| 7 | #4 VL | DIVMTQSPDSLAVSLGERATINCKSSQSLFYSSNQKNFLAW YQQKPGQPPKLLIYWASTRESGVPDRFTGSGSGTDFTLTISS VKAEDLAVYYCQQYYSYPPTFGGGTKLEIK GACATCGTGATGACCCAGTCCCCCGACTCCCTGGCCGTG TCCCTGGGCGAGCGCGCCACCATCAACTGCAAGTCCTCC CAGTCCCTGTTCTACTCCTCCAACCAGAAGAACTTCCTG GCCTGGTACCAGCAGAAGCCCGGCCAGCCCCCCAAGCT GCTGATCTACTGGGCCTCCACCCGCGAGTCCGGCGTGCC CGACCGCTTCACCGGCTCCGGCTCCGGCACCGACTTCAC CCTGACCATCTCCTCCGTGAAGGCCGAGGACCTGGCCGT GTACTACTGCCAGCAGTACTACTCCTACCCCCCCACCTTC GGCGGCGGCACCAAGCTCGAGATAAAA |
| 8 | #4 VL-CDR1 | KSSQSLFYSSNQKNFLA |
| 9 | #4 VL-CDR2 | WASTRES |
| 10 | #4 VL-CDR3 | QQYYSYPPT |
| 11 | #11 VH | QVTLKESGPVLVKPTETLTLTCTVSGFSLSSHGVHWIRQPPG KALEWLAVIWSDGSTTYNSTLKSRLTISKDTSKSQVVLTMT |
| | | NMDPVDTATYYCARHGNYHYNMDYWGQGTLVTVSS |
| 12 | #11 VH | CAGGTGACCCTGAAGGAGTCCGGCCCCGTGCTGGTGAA GCCCACCGAGACCCTGACCCTGACCTGCACCGTGTCCGG CTTCTCCCTGTCCTCCCACGGCGTGCACTGGATCCGCCA GCCCCCCGGCAAGGCCCTGGAGTGGCTGGCCGTGATCTG GTCCGACGGCTCCACCACCTACAACTCCACCCTGAAGTC CCGCCTGACCATCTCCAAGGACACCTCCAAGTCCCAGGT GGTGCTGACCATGACCAACATGGACCCCGTGGACACCGC CACCTACTACTGCGCCCGCCACGGCAACTACCACTACAA CATGGACTACTGGGGCCAGGGCACCCTGGTGACCGTCTC GAGC |
| 13 | #11 VH-CDR1 | SHGVH |
| 14 | #11 VH-CDR2 | VIWSDGSTTYNSTLKS |
| 15 | #11 VH-CDR3 | HGNYHYNMDY |
| 16 | #11 VL | DIQLTQSPSFLSASVGDRVTITCRTSSRVNSGYLHWYQQKP GKAPKLLIYSTSTLASGVPSRFSGSGSGTEFTLTISSLQPEDF ATYYCQQYSSYPWTFGGGTKLEIK |
| 17 | #11 VL | GACATCCAGCTGACCCAGTCCCCCTCCTTCCTGTCCGCCT CCGTGGGCGACCGCGTGACCATCACCTGCCGCACCTCCT CCCGCGTGAACTCCGGCTACCTGCACTGGTACCAGCAGA AGCCCGGCAAGGCCCCCAAGCTGCTGATCTACTCCACCT CCACCCTGGCCTCCGGCGTGCCCTCCCGCTTCTCCGGCT CCGGCTCCGGCACCGAGTTCACCCTGACCATCTCCTCCC TGCAGCCCGAGGACTTCGCCACCTACTACTGCCAGCAGT ACTCCTCCTACCCCTGGACCTTCGGCGGCGGCACCAAGC TCGAGATAAAA |
| 18 | #11 VL-CDR1 | RTSSRVNSGYLH |
| 19 | #11 VL-CDR2 | STSTLAS |
| 20 | #11 VL-CDR3 | QQYSSYPWT |
| 21 | #13 VH | QVQLVQSGSELKKPGASVKVSCKASGYTFTDYSIHWVRQA PGQGLEWMGWINTETGEPTYADDFKGRFVFSLDTSVSTAY LQICSLKAEDTAVYYCSRGPLYRYDGYGLEYWGQGTLVTV SS |
| 22 | #13 VH | CAGGTGCAGCTGGTGCAGTCCGGCTCCGAGCTGAAGAA GCCCGGCGCCTCCGTGAAGGTGTCCTGCAAGGCCTCCGG CTACACCTTCACCGACTACTCCATCCACTGGGTGCGCCA GGCCCCCGGCCAGGGCCTGGAGTGGATGGGCTGGATCA ACACCGAGACCGGCGAGCCCACCTACGCCGACGACTTC AAGGGCCGCTTCGTGTTCTCCCTGGACACCTCCGTGTCC ACCGCCTACCTGCAGATCTGCTCCCTGAAGGCCGAGGAC ACCGCCGTGTACTACTGCTCCCGCGGCCCCCTGTACCGCT ACGACGGCTACGGCCTGGAGTACTGGGGCCAGGGCACC CTGGTGACCGTCTCGAGC |
| 23 | #13 VH-CDR1 | DYSIH |
| 24 | #13 VH-CDR2 | WINTETGEPTYADDFKG |
| 25 | #13 VH-CDR3 | GPLYRYDGYGLEY |
| 26 | #13 VL | DIVMTQSPDSLAVSLGERATINCKASQSVSNDVAWYQQKP GQPPKLLIYYASNRCTGVPDRFSGSGSGTDFTLTISSLQAED VAVYYCHQDYISPYTFGGGTKLEIK |
| 27 | #13 VL | GACATCGTGATGACCCAGTCCCCCGACTCCCTGGCCGTG TCCCTGGGCGAGCGCGCCACCATCAACTGCAAGGCCTCC CAGTCCGTGTCCAACGACGTGGCCTGGTACCAGCAGAA GCCCGGCCAGCCCCCCAAGCTGCTGATCTACTACGCCTC CAACCGCTGCACCGGCGTGCCCGACCGCTTCTCCGGCTC CGGCTCCGGCACCGACTTCACCCTGACCATCTCCTCCCT GCAGGCCGAGGACGTGGCCGTGTACTACTGCCACCAGG ACTACATCTCCCCCTACACCTTCGGCGGCGGCACCAAGC |
| | | TCGAGATAAAA |
| 28 | #13 VL-CDR1 | KASQSVSNDVA |
| 29 | #13 VL-CDR2 | YASNRCT |
| 30 | #13 VL-CDR3 | HQDYISPYT |
| 31 | #14 VH | QVQLQESGPGLVKPSQTLSLTCTVSGGSISSGYYWSWIRQH PGKGLEWIGYISYDGSRYNNPSLKNRVTISVDTSKNQFSLK LSSVTAADTAVYYCAREEYANYFAYWGQGTTVTVSS |
| 32 | #14 VH | CAGGTGCAGCTGCAGGAGAGCGGACCAGGACTGGTGAA GCCTAGCCAGACACTGTCTCTGACCTGCACAGTGAGCGG CGGCTCTATCTCCAGCGGCTACTATTGGTCTTGGATCAGA CAGCACCCAGGCAAGGGCCTGGAGTGGATCGGCTACATC TCCTATGATGGCAGCAGGTATAACAATCCTTCCCTGAAGA ACCGGGTGACCATCTCTGTGGACACATCCAAGAATCAGT TCAGCCTGAAGCTGTCTTCCGTGACCGCCGCTGATACAG CCGTGTACTATTGCGCTCGCGAGGAGTACGCCAACTATTT CGCTTACTGGGGCCAGGGCACCACAGTGACCGTGAGCTC T |
| 33 | #14 VH-CDR1 | SGYYWS |
| 34 | #14 VH-CDR2 | YISYDGSRYNNPSLKN |
| 35 | #14 VH-CDR3 | EEYANYFAY |
| 36 | #14 VL | DIVMTQSPDSLAVSLGERATINCKSSQSLFYSSNQKNFLAW YQQKPGQPPKLLIYWASTRESGVPDRFTGSGSGTDFTLTISS VKAEDLAVYYCQQYYSYPPTFGQGTKLEIK |
| 37 | #14 VL | GATATCGTGATGACCCAGTCTCCTGACTCCCTGGCCGTGA GCCTGGGCGAGAGAGCTACAATCAACTGTAAGTCCAGCC |
| | | A GTCTCTGTTCTACTCTTCCAACCAGAAGAATTTTCTGGCC TGGTATCAGCAGAAGCCCGGCCAGCCCCCTAAGCTGCTG A TCTACTGGGCTAGCACCAGAGAGTCTGGAGTGCCTGACC G CTTCACCGGATCCGGAAGCGGAACAGACTTCACCCTGAC A ATCAGCTCTGTGAAGGCCGAGGATCTGGCCGTGTACTATT GCCAGCAGTACTATTCTTATCCACCCACCTTCGGCCAGGG CACAAAGCTCGAGATCAAG |
| 38 | #14 VL-CDR1 | KSSQSLFYSSNQKNFLA |
| 39 | #14 VL-CDR2 | WASTRES |
| 40 | #14 VL-CDR3 | QQYYSYPPT |

**Table 4. Heavy and light chain amino acid sequences of #14 antibody**

| SEQ ID NO: | Name | Sequence |
|---|---|---|
| 41 | #14 HC | QVQLQESGPGLVKPSQTLSLTCTVSGGSISSGYYWSWIRQH PGKGLEWIGYISYDGSRYNNPSLKNRVTISVDTSKNQFSLK LSSVTAADTAVYYCAREEYANYFAYWGQGTTVTVSSASTK GPSVFPLAPSSKSTSGGTAALGCLVKDYFPEPVTVSWNSGA LTSGVHTFPAVLQSSGLYSLSSVVTVPSSSLGTQTYICNVNH KPSNTKVDKKVEPKSCDKTHTCPPCPAPELLGGPSVFLFPP KPKDTLMISRTPEVTCVVVDVSHEDPEVKFNWYVDGVEV HNAKTKPREEQYASTYRVVSVLTVLHQDWLNGKEYKCKV SNKALPAPIEKTISKAKGQPREPQVYTLPPSREEMTKNQVS LTCLVKGFYPSDIAVEWESNGQPENNYKTTPPVLDSDGSFF LYSKLTVDKSRWQQGNVFSCSVMHEALHNHYTQK SLSLSPGK |
| 42 | #14 LC | DIVMTQSPDSLAVSLGERATINCKSSQSLFYSSNQKNFLAW YQQKPGQPPKLLIYWASTRESGVPDRFTGSGSGTDFTLTISS |
| | | VKAEDLAVYYCQQYYSYPPTFGQGTKLEIKRTVAAPSVFIF PPSDEQLKSGTASVVCLLNNFYPREAKVQWKVDNALQSG NSQESVTEQDSKDSTYSLSSTLTLSKADYEKHKVYACEVTH QGLSSPVTKSFNRGEC |

### Example 1: Preparation of mouse anti-SIRPα monoclonal antibodies

This example describes the preparation of mouse anti-human SIRPα monoclonal antibodies using hybridoma technology. The extracellular region of SIRPα protein (Uniprot: P78324) was expressed as the immunogen. Human IgG1 Fc tag was fused to the C terminus of the amino acid sequence (Glu31-Arg370) of the SIRPα protein extracellular region. The gene was then cloned into vector V152 (supplied by Huabio) to produce vector V152-SIRPα ECD-Fc. The vector was transiently transfected into 293 cells (supplied by ATCC). After five days, the cell culture supernatant was collected and purified by protein A (manufacturer: Solarbio, Cat. No. I8090). To prepare mouse anti-human SIRPα monoclonal antibodies, 4-week-old BAL B/c mice (supplied by Huabio) were immunized with 100 µg of SIRPα protein. On days 14 and 28 after the first immunization, the immunized mice were re-immunized with 50 µg of SIRPα protein. The serum titer in the immunized mice was measured by ELISA. 96-well plates were coated with SIRPα (1 µg/mL) in PBS (manufacturer: ZSGB-BIO, Cat. No. ZLI-9062) at 4 °C overnight. Plates were blocked with 1% BSA-PBS blocking solution at 37 °C for 1 h and washed with PBST. Serum dilutions from immunized mice were added to the plates and the plates were incubated for 1 h at 37 °C. The plates were washed and HRP-labeled goat anti-mouse IgG (manufacturer: Abcam, Cat. No. Ab205719, 1:10000 dilution) was added. After a 0.5 h incubation at 37 °C, the plates were washed and TMB solution (manufacturer: InnoReagents, Cat. No. TMB-S-00) was added. After a 5 min incubation at room temperature in the dark, the reaction was terminated by adding 2 N H₂SO₄. Absorbance was read at 450 nm on a microplate reader. Mice with sufficient titers of anti-SIRPα antibody were immunized with 50 µg of SIRPα protein as a booster dose on day 42. After 3-5 days, the mice were sacrificed. The spleen cells were collected, washed 2-3 times with IMDM basal medium (manufacturer: BasalMedia, Cat. No. L610KJ) by centrifugation, and then mixed with mouse myeloma cells SP2/0 (supplied by Huabio) in a ratio of 1:1. PEG (manufacturer: Roche, Cat. No. 25771700) was added to the mixed cells, and the mixture was gently stirred and let stand at 37 °C for 30 s. The fused cells were diluted in IMEM selection medium containing 15% fetal bovine serum (manufacturer: Tianhang Biotechnology, Cat. No. 11011-8611) and 1× HAT (manufacturer: Sigma, Cat. No. H0262-1VL), added to a 96-well cell culture plate at 200 µL/well, and incubated in a 5% CO₂ incubator at 37 °C. After 10-14 days, the anti-SIRPα antibody in the supernatant of the hybridoma cells was detected by ELISA. Eleven different hybridoma clones, 10F11-5-6, 1B6-1-1, 27A11-1-8, 14B11-5-4, 31D4-4-5, 7C2-3-8, 30C1-6-4, 4A3-5-1, 6H1-8-6, 9A12-5 and 10F4-15, were identified and subjected to further analysis.

### Example 2: Binding of murine antibodies to SIRPα

The binding capacity of the murine antibodies to SIRPα protein was detected by ELISA. The specific method is as described in Example 1. The murine antibodies were 3-fold serially diluted with PBS from 1 µg/mL, and a total of seven concentration gradients were prepared. The results are shown in Table 5.

**Table 5: Binding activity (EC₅₀) of murine monoclonal antibodies to SIRPα**

| Antibody No. | Clone | ELISA EC50 (pM) |
|---|---|---|
| mAb#4 | 10F11-5-6 | 4.22 |
| mAb#6 | 1B6-1-1 | 9.15 |
| mAb#11 | 27A11-1-8 | 5.47 |
| mAb#12 | 14B11-5-4 | 8.67 |
| mAb#13 | 31D4-4-5 | 6.77 |
| mAb#14 | 7C2-3-8 | 5.69 |
| mAb#15 | 30C1-6-4 | 10.31 |
| mAb#16 | 4A3-5-1 | 10.64 |
| mAb#17 | 6H1-8-6 | 11.93 |
| mAb#18 | 9A12-5 | 8.05 |
| mAb#19 | 10F4-15 | 8.66 |

The binding of murine monoclonal antibodies to membrane SIRPα on human myeloid leukemia monocytes (THP-1, supplied by Cell Bank of Chinese Academy of Sciences) endogenously expressing SIRPα was analyzed by flow cytometry (FACS). THP-1 cells were co-incubated with murine monoclonal antibodies at various concentrations (3-fold serially diluted from 1 µg/mL, seven concentration gradients in total) for 30 min at 4 °C. The cells were washed twice before FITC-labeled goat anti-mouse IgG (manufacturer: Jackson, Cat. No. 115-095-003, 1:1000 dilution) was added and then incubated at 4 °C for 30 min in the dark . After being washed twice, the cells were detected on flow cytometer BD C6. The results are shown in Table 6.

**Table 6: (EC₅₀) for binding of murine monoclonal antibodies to THP-1 cells**

| Antibody No. | Clone | FACS EC50 (pM) |
|---|---|---|
| mAb#4 | 10F11-5-6 | 139.22 |
| mAb#6 | 1B6-1-1 | 231.81 |
| mAb#11 | 27A11-1-8 | 125.84 |
| mAb#12 | 14B11-5-4 | 211.43 |
| mAb#13 | 31D4-4-5 | 144.27 |
| mAb#14 | 7C2-3-8 | 128.72 |
| mAb#15 | 30C1-6-4 | 128.70 |
| mAb#16 | 4A3-5-1 | 232.4 |
| mAb#17 | 6H1-8-6 | 544.21 |
| mAb#18 | 9A12-5 | 212.34 |
| mAb#19 | 10F4-15 | 220.11 |

### Example 3: Blocking effect of murine anti-SIRPα antibodies on binding of SIRPα to CD47

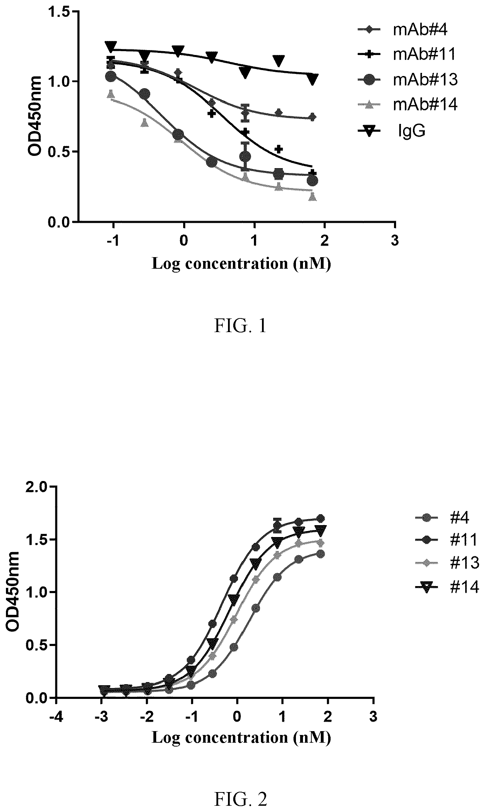

According to the results of Example 2, combining the binding ability of mouse monoclonal antibody to SIRPα and the binding ability to THP-1 cells, mAb#4, mAb#11, mAb#13 and mAb#14 are the molecules with the best activity. Therefore, mAb#4, mAb#11, mAb#13 and mAb#14 were selected for determining whether these murine mAbs could block the binding of SIRPα to CD47. 96-well plates were coated with CD47-Fc (manufacturer: Acrobiosystems, Cat. No. CD7-H5256) at 4 °C overnight. Plates were washed and blocked with 1% BSA-PBST blocking solution. After a 1 h incubation at 37 °C, plates were washed, and antibodies of different concentrations (3-fold diluted from 66.7 nM, seven concentration gradients in total) and biotin-labeled SIRPα (manufacturer: Acrobiosystems, Cat. No. CDA-H82F2) were added to the plates. After a 1 h incubation at 37 °C, plates were washed and HRP-labeled streptavidin (manufacturer: Abcam, Cat. No.: ab7403) was added. After a 0.5 h incubation at 37 °C, plates were washed and TMB solution was added. After a 0.5 h incubation at room temperature in the dark, the reaction was terminated by adding 2 N H₂SO₄. Absorbance was read at 450 nm on a microplate reader. IgG (manufacturer: GenScript) was used as the control. The results, as shown in FIG. 1, demonstrate that these murine antibodies can all inhibit the binding of SIRPα to CD47 to various extents.

### Example 4: Humanization of murine antibodies

Based on the results of Example 2 and Example 3, mAb#4, mAb#11, mAb#13 and mAb#14 were selected for humanization. The heavy and light chain variable regions of these antibodies were compared to the available database of human IgG gene sequences to identify the optimal human germline Ig gene sequence matches. Specifically, the human germline IgG heavy chains selected for mAb#4, mAb#11, mAb#13 and mAb#14 were IGHV1-69-2*01, IGHV2-26^{∗}01, IGHV7-4-1^{∗}01 and IGHV4-31*02, respectively, and the human germline IgG light chains selected for mAb#4, mAb#11, mAb#13 and mAb#14 were IGKV4-1*01, IGKV1-9*01, IGKV4-1*01 and IGKV4-1*01, respectively. The heavy chain CDRs of mAb#4, mAb#11, mAb#13 and mAb#14 were grafted into the framework region of the human germline IgG heavy chains, and the light chain CDRs of mAb#4, mAb#11, mAb#13 and mAb#14 were grafted into the framework region of human germline IgG light chains. The new humanized molecules were designated #4, #11, #13 and #14, respectively. The sequences of the heavy chain variable region, light chain variable region and CDR regions of the constructed humanized antibodies are shown in Table 3. The heavy and light chain sequences of #14 antibody are shown in Table 4. The heavy and light chain constant regions of #4, #11 and #13 antibodies are consistent with those of #14 antibody. The complete amino acid sequences of the heavy and light chains of #4, #11, and #13 antibodies are known to those skilled in the art based on the information disclosed above.

The antibodies disclosed herein were expressed in 293 cells (supplied by ATCC) and purified. The coding sequences of the antibody heavy and light chains were cloned into V152 vector (supplied by Huabio). The V152 vector carrying the coding sequences of the antibody heavy and light chains was transferred into 293 cells using transfection reagent PEI (manufacturer: Polyscience, Cat. No. 23966-2). Plasmid DNA and the transfection reagent were prepared in a biosafety cabinet. The plasmid DNA and PEI (mass ratio = 0.15:1.75) were mixed uniformly and then let stand for 10 min. The mixture was added to 293 cells gently and mixed gently. After the cells were cultured at 37 °C and 5% CO₂ for 5 days, the cell culture supernatant was centrifuged at 3000 rpm for 10 min. The supernatant was purified with Protein A (manufacturer: Solarbio, Cat. No. I8090) to make the antibody purity greater than 95%.

### Example 5: Binding of humanized antibodies to SIRPα protein

The binding of the humanized antibodies to SIRPα protein was detected by ELISA. 96-well plates were coated with SIRPα (manufacturer: Acrobiosystems, Cat. No. SIA-H5225) (1 µg/mL) in PBS at 4 °C overnight. The plates were then blocked with 1% BSA-PBS blocking solution for 1 h at 37 °C. Plates were washed with PBST. The humanized antibodies were diluted to different concentrations (3-fold serially diluted from 66.7 nM, 11 concentration gradients in total) and added to the plates. After a 1 h incubation at 37 °C, plates were washed and HRP-labeled goat anti-human IgG (manufacturer: Abcam, Cat. No. Ab98595, 1:10000 dilution) was added. After a 0.5 h incubation at 37 °C, plates were washed and TMB solution was added. After a 5 min incubation at room temperature in the dark, the reaction was terminated by adding 2 N H₂SO₄. Absorbance was read at 450 nm on a microplate reader. The results are shown in FIG. 2. The (EC₅₀) values for binding to SIRPα of #4, #11, #13 and #14 antibodies were 1.833 nM, 0.4642 nM, 0.9517 nM and 0.6831 nM, respectively, indicating that these humanized molecules all have a high binding activity to SIRPα.

### Example 6: Blocking effect of humanized anti-SIRPα antibodies on binding of SIRPα to CD47

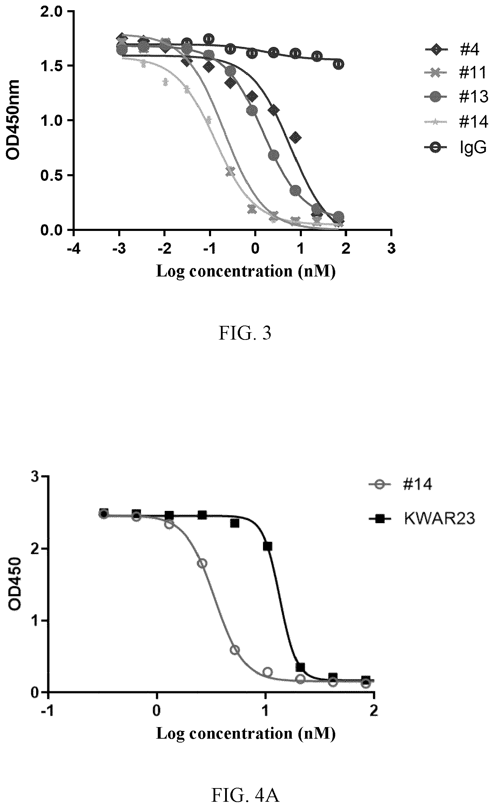

The blocking effect of the humanized anti-SIRPα antibodies #4, #11, #13 and #14 on the binding of SIRPα to CD47 was tested as described in Example 3. The antibodies were 3-fold serially diluted from 66.7 nM and a total of 11 concentration gradients were prepared. The results are shown in FIG. 3. The IC₅₀ values of #4, #11, #13 and #14 antibodies were 6.03 nM, 0.2086 nM, 1.5 nM and 0.1315 nM, respectively, showing that all these humanized antibodies could effectively block the binding of SIRPα to CD47, with #11 and #14 antibodies having stronger blocking effect than the other two humanized antibodies.

KWAR23, 18D5 and 1H9 were used as controls. KWAR23 was prepared according to the sequences disclosed in US2018037652 (SEQ ID NO: 1 and SEQ ID NO: 2), 18D5 according to the sequences disclosed in US20190127477 (SEQ ID NO: 24 and SEQ ID NO: 31), and 1H9 according to the sequences disclosed in US20190119396 (SEQ ID NO: 7 and SEQ ID NO: 8). Since the human SIRPα protein is highly polymorphic in the extracellular region, and the common genotypes include V1, V2 and V8, the blocking effects of #14 antibody and the above control antibodies on the binding of common SIRPα genotypes to CD47 were further compared (in other examples, the SIRPα proteins with no specified genotype are all of V1 genotype).

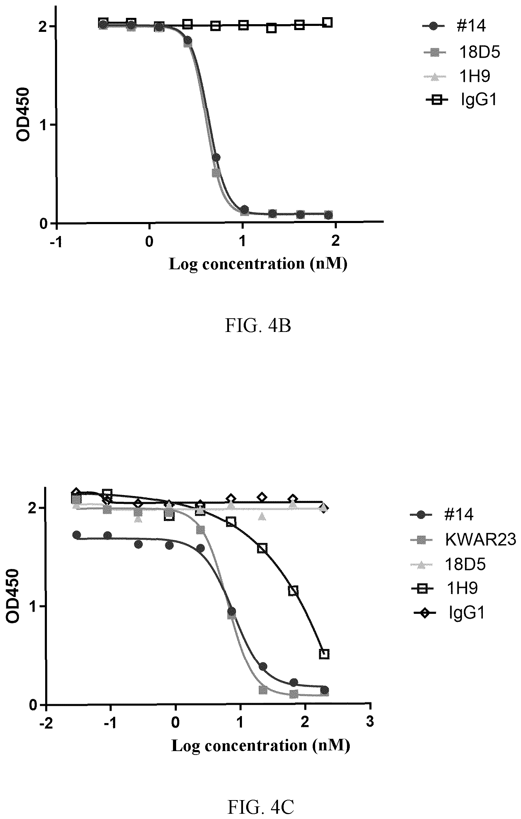

The blocking effects of different antibodies on the binding of SIRPα (V1) to CD47 were tested as described in Example 3. The antibodies were 2-fold serially diluted from 83.4 nM and a total of nine concentration gradients were prepared. The results are shown in FIG. 4A. The IC₅₀ value of #14 antibody was 3.404 nM and the IC₅₀ value of KWAR23 was 13.54 nM, indicating that #14 antibody has a stronger blocking effect on the binding of SIRPα (V1) to CD47 than KWAR23. In FIG. 4B, the IC₅₀ values of #14 antibody, 18D5 and 1H9 were 4.4 nM, 4.1 nM and 4.2 nM, respectively, indicating that the blocking activities of #14 antibody, 18D5 and 1H9 on the binding of SIRPα (V1) to CD47 were similar.

The blocking effects of different antibodies on the binding of SIRPα (V2) to CD47 was determined by the method described in Example 3, where the biotin-labeled SIRPα used was SIRPα (V2) (manufacturer: Acrobiosystems, Cat. No. SI2-H82W8). The antibodies were 3-fold serially diluted from 200 nM and a total of nine concentration gradients were prepared. The results are shown in FIG. 4C. #14 antibody, KWAR23 and 1H9 can block the binding of SIRPα (V2) to CD47, while 18D5 cannot block the binding of SIRPα (V2) to CD47. The blocking activity of #14 antibody is stronger than that of 1H9. The IC₅₀ value of #14 antibody was 7.8 nM and the IC₅₀ value of KWAR23 was 6.4 nM, indicating that #14 antibody and KWAR23 have similar blocking activities against SIRPα (V2).

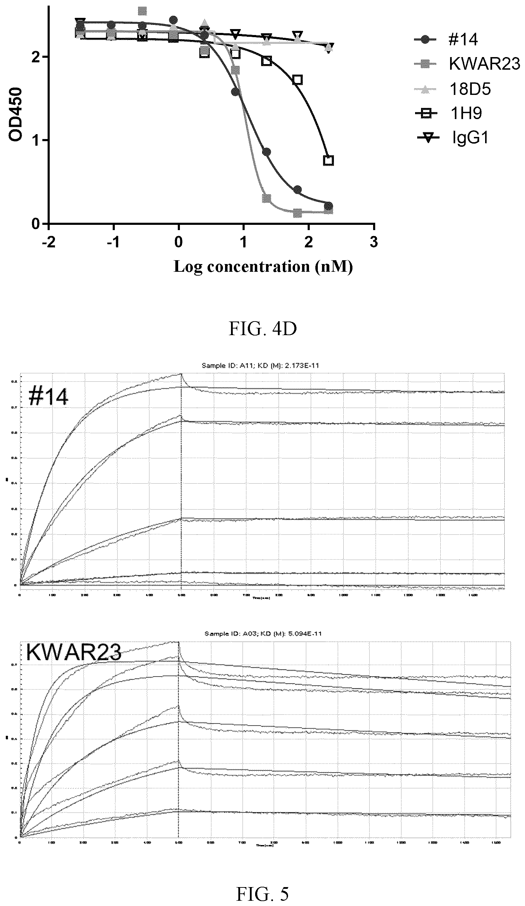

The blocking effects of different antibodies on the binding of SIRPα (V8) to CD47 was determined by the method described in Example 3, where the biotin-labeled SIRPα used was SIRPα (V8) (manufacturer: Acrobiosystems, Cat. No. SI8-H82W6). The antibodies were 3-fold serially diluted from 200 nM and a total of nine concentration gradients were prepared. The results are shown in FIG. 4D. #14 antibody, KWAR23 and 1H9 can block the binding of SIRPα (V8) to CD47, while 18D5 cannot block the binding of SIRPα (V8) to CD47. The blocking activity of #14 antibody is stronger than that of 1H9. The IC₅₀ value of #14 antibody was 11.5 nM and the IC₅₀ value of KWAR23 was 10.8 nM, indicating that #14 antibody and KWAR23 have similar blocking activities against SIRPα (V8).

In accordance with the above results, #14 antibody can block the binding of common SIRPα genotypes (V1, V2 and V8) to CD47, and has a stronger blocking activity for SIRPα (V1) than KWAR23 and a stronger blocking activity for SIRPα (V2) and SIRPα (V8) than 18D5 and 1H9.

### Example 7: Binding kinetics of antibodies to SIRPα

Antibody-to-antigen binding kinetic was determined using Octet Red96 system (manufacturer: ForteBio) equipped with an H1S1K biosensor (manufacturer: ForteBio). The biosensor was directly used to capture the antigen, and then immersed in the analyte sample (antibody) right after that. The experiment consisted of five procedures: 1, baseline (100 s), 2, loading (capturing the antigen SIRPα) (500 s), 3, baseline (100 s), 4, association (binding to the antibody) (500 s), 5, dissociation (antibody dissociation) (1000 s). After completion of the test, the sensor was regenerated by alternating soaking in regeneration buffer (glycine, pH 1.5) and neutralization buffer (PBS) for 5 seconds, and a total of five cycles were performed to regenerate the sensor. The operating buffer in this experiment was PBS.

Sample treatment: the antigen SIRPα protein (manufacturer: Acrobiosystems, Cat. No. SIA-H5225) was diluted to a working concentration of 2.5 µg/mL using the operating buffer and the analyte samples (#14 antibody and KWAR23) were serially diluted to five working concentrations: 1 µg/mL, 0.5 µ/mL, 0.25 µg/mL, 0.125 µg/mL and 0.0625 µg/mL. For data analysis, the response signal values (coupled analyte sample signal subtracted by blank analyte sample signal) were calculated using Octet Data Analysis (version 7.0 or the latest) and the data was fitted using a 1:1 association model.

The fitting diagram of the binding kinetics is shown in FIG. 5, and the association, dissociation and equilibrium constants are shown in Table 7. It can be seen that #14 antibody has a very high affinity, which is comparable to that of the positive control antibody KWAR23.

**Table 7: Binding kinetics of #14 antibody and KWAR23 to SIRPα**

| Sample name | Kon | Koff | KD (M) |
|---|---|---|---|
| #14 | 1.33×10⁶ | 2.89×10⁻⁵ | 2.173×10⁻¹¹ |
| KWAR23 | 2.90×10⁶ | 1.48×10⁻⁴ | 5.094×10⁻¹¹ |

### Example 8: Promotion effect of anti-SIRPα antibodies disclosed herein on phagocytosis of tumor cells by macrophages

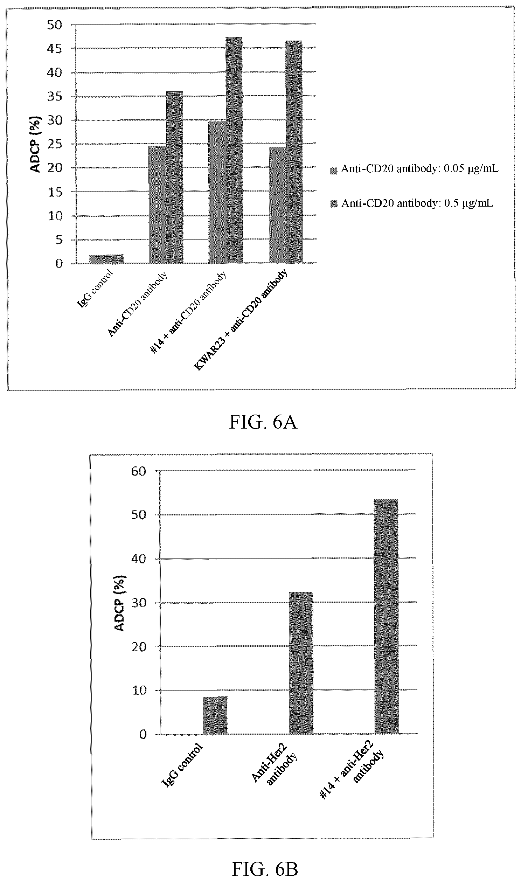

PBMC cells (manufacturer: Allcells, Cat. No. PB004F-C) were cultured in 1640 basal medium (manufacturer: Gibco, Cat. No. 22400-089) at 37 °C and 5% CO₂ for 2-3 h. Nonadherent cells were removed by pipette, and an induction medium (80 ng/mL M-CSF) was added for induction. Change fresh medium containing sufficient cytokines every 3 days. The cells were cultured for 7 days to obtain macrophages. Raji cells (supplied by Cell Bank of Chinese Academy of Sciences) were fluorescently labeled according to the instruction of CFSE reagent (manufacturer: Abcam, Cat. No. AB113853). The labeled Raji target cells and the above differentiated macrophages were mixed evenly at a ratio of 3:1, and an anti-CD20 antibody (zuberitamab, CAS RN: 2251143-19-6, WHO Drug Information, Vol. 33, No.4, 2019, 914-915; concentration: 0.05 µg/mL, 0.5 µg/mL; manufacturer: BioRay), a combination of the anti-CD20 antibody (concentration: 0.05 µg/mL, 0.5 µg/mL) and #14 antibody (concentration: 10 µg/mL), or a combination of the anti-CD20 antibody (concentration: 0.05 µg/mL, 0.5 µg/mL) and KWAR23 antibody (concentration: 10 µg/mL) was added. The cells were cultured at 37 °C and 5% CO₂ for 4 h. Then cells were washed with PBS, and anti-CD14-APC (manufacturer: BD Biosciences, Cat. No. 555399) was added. After a 30 min incubation at 4 °C in the dark, cells were washed and analyzed by flow cytometry. The phagocytosis rate (ADCP) was calculated according to the following formula: phagocytosis rate (%) = ratio of (APC + CFSE) positive cells/ratio of APC positive cells × 100%. FIG. 6A shows that #14 antibody can enhance the phagocytosis of Raji cells induced by the anti-CD20 antibody.

The promotion effect of the combination of #14 antibody and the anti-Her2 antibody on macrophage phagocytosis of SK-BR-3 cells overexpressing Her2 was also investigated. According to the method described above, the labeled SK-BR-3 cells (supplied by Cell Bank of Chinese Academy of Sciences) and the differentiated macrophages were mixed evenly at a ratio of 2:1, and an anti-Her2 antibody (concentration: 0.5 µg/mL; manufacturer: BioRay) or a combination of the anti-Her2 antibody (concentration: 0.5 µg/mL) and #14 antibody (concentration: 10 µg/mL) was added. The cells were cultured at 37 °C and 5% CO₂ for 2 h. The phagocytosis rate was analyzed and calculated by flow cytometry. FIG. 6B shows that #14 antibody can enhance the phagocytosis of SK-BR-3 cells induced by the anti-Her2 antibody.

### Example 9: The anti-SIRPα antibody disclosed herein induces the endocytosis of SIRPα on cell surface

THP-1 cells (supplied by Cell Bank of Chinese Academy of Sciences) were co-incubated with 10 µg/mL antibody at 4 °C for 30 min. The cells were washed twice, and divided into two parts. One part was incubated at 37 °C for 4 h, and the other part was further incubated at 4 °C as a control. The cells were washed twice and FITC-labeled goat anti-human IgG (manufacturer: Abcam, Cat. No. ab97224) was added. After a 30 min incubation at 4 °C in the dark, the cells were washed and then analyzed by flow cytometry. The endocytosis rate was calculated from the decrease in cell surface fluorescence at 37 °C versus 4 °C. The endocytosis rate was calculated according to the following formula: endocytosis rate (%) = (fluorescence intensity MFI at 4 °C - fluorescence intensity MFI at 37 °C)/fluorescence intensity MFI at 4 °C × 100%.

Table 8 shows that the endocytosis rate for #14 antibody was 52.9%, while the endocytosis rate for KWAR23 was 34.7%, indicating that #14 antibody is more effective in reducing the expression level of cell surface SIRPα.

**Table 8: Endocytosis of SIRPα on THP-1 cell membrane induced by #14 and KWAR23**

| Sample | Endocytosis rate (%) |
|---|---|
| #14 | 52.9 |
| KWAR23 | 34.7 |

### Example 10: Tumor growth inhibition induced by the anti-SIRPα antibody disclosed herein

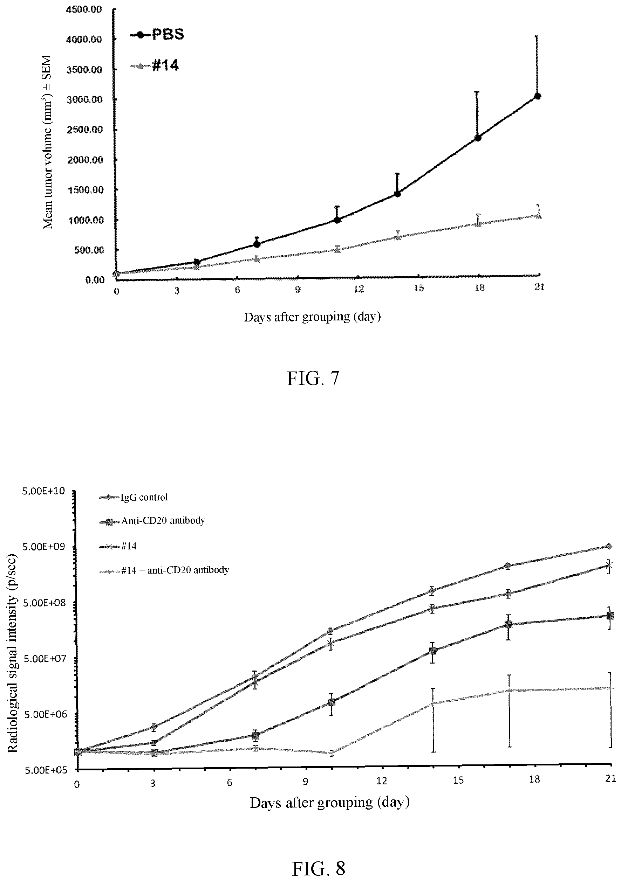

The anti-tumor efficacy of #14 antibody was evaluated in an hCD47-MC38 mouse colon cancer model. hCD47-MC38 cells (supplied by Shanghai Model Organisms) were inoculated into hSIRPα/hCD47 dual humanized C57BL/6 mice (supplied by Shanghai Model Organisms). When the tumor volume reached about 150 mm³, the mice were administered with #14 antibody (200 µg/mouse) by intraperitoneal injection twice a week, and the control group received equivalent amounts of PBS. The results are shown in FIG. 7. #14 antibody can continuously inhibit the tumor growth as compared with the control group.

The anti-tumor efficacy of the combination of #14 antibody and an anti-CD20 antibody (zuberitamab, supplied by BioRay) in a lymphoma model was also evaluated. Raji-Luc cells (supplied by Biocytogen) were inoculated into B-NDG-hSIRPα mice (supplied by Biocytogen) via tail veins. When the average tumor imaging signal intensity reached about 1 × 10⁶ p/sec, the mice were administered with IgG control (supplied by Biocytogen, 200 µg/mouse), #14 antibody alone (200 µg/mouse), anti-CD20 antibody alone (2 µg/mouse) or #14 antibody (200 µg/mouse) plus anti-CD20 antibody (2 µg/mouse) at the designated doses. As shown in FIG. 8, #14 antibody and the anti-CD20 antibody have synergistic anti-tumor efficacy.

## Claims

1. An anti-SIRPα antibody or an antigen-binding fragment thereof, comprising: a heavy chain variable region comprising three CDRs, VH CDR1, VH CDR2 and VH CDR3, and a light chain variable region comprising three CDRs, VL CDR1, VL CDR2 and VL CDR3, wherein
the VH CDR1 comprises or consists of an amino acid sequence set forth in SEQ ID NO: 3, 13, 23 or 33, the VH CDR2 comprises or consists of an amino acid sequence set forth in SEQ ID NO: 4, 14, 24 or 34, the VH CDR3 comprises or consists of an amino acid sequence set forth in SEQ ID NO: 5, 15, 25 or 35, the VL CDR1 comprises or consists of an amino acid sequence set forth in SEQ ID NO: 8, 18, 28 or 38, the VL CDR2 comprises or consists of an amino acid sequence set forth in SEQ ID NO: 9, 19, 29 or 39, and the VL CDR3 comprises or consists of an amino acid sequence set forth in SEQ ID NO: 10, 20, 30 or 40.

2. The anti-SIRPα antibody or the antigen-binding fragment thereof according to claim 1, comprising VH CDR1, VH CDR2 and VH CDR3, and VL CDR1, VL CDR2 and VL CDR3, wherein
the amino acid sequence of the VH CDR1 is set forth in SEQ ID NO: 3;
the amino acid sequence of the VH CDR2 is set forth in SEQ ID NO: 4;
the amino acid sequence of the VH CDR3 is set forth in SEQ ID NO: 5;
the amino acid sequence of the VL CDR1 is set forth in SEQ ID NO: 8;
the amino acid sequence of the VL CDR2 is set forth in SEQ ID NO: 9; and
the amino acid sequence of the VL CDR3 is set forth in SEQ ID NO: 10;
or,
the amino acid sequence of the VH CDR1 is set forth in SEQ ID NO: 13;
the amino acid sequence of the VH CDR2 is set forth in SEQ ID NO: 14;
the amino acid sequence of the VH CDR3 is set forth in SEQ ID NO: 15;
the amino acid sequence of the VL CDR1 is set forth in SEQ ID NO: 18;
the amino acid sequence of the VL CDR2 is set forth in SEQ ID NO: 19; and
the amino acid sequence of the VL CDR3 is set forth in SEQ ID NO: 20;
or,
the amino acid sequence of the VH CDR1 is set forth in SEQ ID NO: 23;
the amino acid sequence of the VH CDR2 is set forth in SEQ ID NO: 24;
the amino acid sequence of the VH CDR3 is set forth in SEQ ID NO: 25;
the amino acid sequence of the VL CDR1 is set forth in SEQ ID NO: 28;
the amino acid sequence of the VL CDR2 is set forth in SEQ ID NO: 29; and
the amino acid sequence of the VL CDR3 is set forth in SEQ ID NO: 30;
or,
the amino acid sequence of the VH CDR1 is set forth in SEQ ID NO: 33;
the amino acid sequence of the VH CDR2 is set forth in SEQ ID NO: 34;
the amino acid sequence of the VH CDR3 is set forth in SEQ ID NO: 35;
the amino acid sequence of the VL CDR1 is set forth in SEQ ID NO: 38;
the amino acid sequence of the VL CDR2 is set forth in SEQ ID NO: 39; and
the amino acid sequence of the VL CDR3 is set forth in SEQ ID NO: 40.

3. The anti-SIRPα antibody or the antigen-binding fragment thereof according to claim 1 or 2, wherein the amino acid sequence of the heavy chain variable region is set forth in SEQ ID NO: 1, 11, 21 or 31, or has at least 90%, at least 95%, at least 96%, at least 97%, at least 98% or at least 99% sequence identity thereto; the amino acid sequence of the light chain variable region is set forth in SEQ ID NO: 6, 16, 26 or 36, or has at least 90%, at least 95%, at least 96%, at least 97%, at least 98% or at least 99% sequence identity thereto.

4. The anti-SIRPα antibody or the antigen-binding fragment thereof according to any one of claims 1-3, further comprising one or more of a heavy chain constant region, a light chain constant region and an Fc region; preferably, the light chain constant region is a λ chain or a κ chain constant region; more preferably, the antibody or the antigen-binding fragment thereof is of IgG1, IgG2, IgG3 or IgG4 type; even more preferably, the antibody or the antigen-binding fragment thereof is a chimeric antibody or a humanized antibody, or an antigen-binding fragment thereof.

5. A nucleic acid molecule, comprising nucleotides encoding the antibody or the antigen-binding fragment thereof according to any one of claims 1-4; preferably, the nucleic acid molecule encodes the heavy chain variable region and/or the light chain variable region of the antibody or the antigen-binding fragment thereof; more preferably, the nucleic acid molecule encodes the heavy chain variable region, and the corresponding nucleotide sequence is set forth in SEQ ID NO: 2, 12, 22 or 32, or has at least 90%, at least 95%, at least 96%, at least 97%, at least 98% or at least 99% sequence identity thereto; or the nucleic acid molecule encodes the light chain variable region, and the corresponding nucleotide sequence is set forth in SEQ ID NO: 7, 17, 27 or 37, or has at least 90%, at least 95%, at least 96%, at least 97%, at least 98% or at least 99% sequence identity thereto.

6. A biomaterial, wherein the biomaterial is:
(1) a vector, a host cell or a microorganism comprising the nucleic acid molecule according to claim 5; or
(2) an expression product, a suspension or a supernatant of the above (1).

7. A composition, comprising the antibody or the antigen-binding fragment thereof according to any one of claims 1-4; preferably, the composition is a pharmaceutical composition further comprising a pharmaceutically acceptable carrier.

8. A method for preparing the antibody or the antigen-binding fragment thereof according to any one of claims 1-4, comprising: culturing the host cell according to claim 6 to express the antibody or the antigen-binding fragment, and isolating the antibody or the antigen-binding fragment.

9. Use of the antibody or the antigen-binding fragment thereof according to any one of claims 1-4, the nucleic acid molecule according to claim 5, the biomaterial according to claim 6 or the composition according to claim 7 in preparing a medicament for treating a tumor; preferably, the tumor is an SIRPα-positive tumor; more preferably, the tumor is a hematological tumor or a solid tumor, such as leukemia, lymphoma, bladder cancer, breast cancer, head and neck cancer, gastric cancer, melanoma, pancreatic cancer, colorectal cancer, esophageal cancer, liver cancer, kidney cancer, lung cancer, prostate cancer, ovarian cancer, thyroid cancer, neuroglioma and other solid tumors.

10. Use of the antibody or the antigen-binding fragment thereof according to any one of claims 1-4, the nucleic acid molecule according to claim 5, the biomaterial according to claim 6 or the composition according to claim 7 in preparing a formulation that blocks the binding of SIRPα to CD47.

11. Use of the antibody or the antigen-binding fragment thereof according to any one of claims 1-4, the nucleic acid molecule according to claim 5, the biomaterial according to claim 6 or the composition according to claim 7 in combination with one or more additional cancer therapeutic agents in preparing a medicament for treating a tumor; preferably, the tumor is an SIRPα-positive tumor; preferably, the additional cancer therapeutic agent includes, but is not limited to, a chemotherapeutic agent, a radiotherapeutic agent and a biomacromolecule drug; more preferably, the biomacromolecule drug is a monoclonal antibody drug targeting a tumor cell surface antigen, such as an anti-CD20 antibody, cetuximab or trastuzumab; more preferably, the anti-CD20 antibody is zuberitamab or rituximab.
